# EUROPEAN PATENT APPLICATION

(11) **EP 4 047 616 A1**
(43) Date of publication of application: **24.08.2022**
(21) Application number: 21157765.5
(22) Date of filing: 18.02.2021
(51) Int. Cl.: G16H 40/63, G16H 50/20

(54) **A PLANNING SYSTEM FOR PLANNING BREAST FEEDING OR MILK EXPRESSION**

(71) Applicant: Koninklijke Philips N.V., 5656 AG Eindhoven (NL)
(72) Inventor: VAN BENTUM, Jesper William, 5656 AE Eindhoven (NL); MARGARITO, Jenny, 5656 AE Eindhoven (NL); RABOTTI, Chiara, 5656 AE Eindhoven (NL); LONG, Xi, 5656 AE Eindhoven (NL)
(74) Representative: Philips Intellectual Property & Standards

(57) **Abstract**

A planning system is provided for planning breast feeding or milk expression. An input receives first milk flow information from breast pumping sessions and second milk flow information from breast feeding sessions. The first and second milk flow information are processed to derive a schedule, wherein the schedule specifies a combination of future breast pumping and breast feeding sessions. In this way, the system assists a mother in planning her milk production routine to best fit with her schedule as well as providing the best nutrition for the infant.

## Description

### FIELD OF THE INVENTION

This invention relates to a planning system and method for breast feeding or expression using a breast pump.

### BACKGROUND OF THE INVENTION

Breast feeding is the most natural way of infant feeding and it is associated with many short term and long term medical and neurodevelopmental advantages. It is well known that breast feeding drastically reduces the risk of sudden infant death syndrome, respiratory tract infections, gastrointestinal tract infections, necrotizing enterocolitis, allergic diseases, inflammatory bowel disease, celiac disease, obesity, childhood leukemia and lymphoma. It also improves neurodevelopmental outcomes. Consequently, the American Academy of Pediatrics strongly recommends mothers to breastfeed for 1 year or longer.

Besides the clear health benefits, breast feeding also improves maternal-infant bonding. It is therefore most natural that women want to breast feed. However, breast feeding is also associated with a variety of problems. Some mothers are not able to breast feed, breast feeding can be painful, and mothers find it often challenging to assess whether their baby is provided with sufficient milk.

To provide greater insights into the milk flow of the lactating human breast, different technological approaches have been developed. It has for example been proposed in US 2009/0054771 to use Doppler ultrasound sensors, integrated into a wearable brassiere, to monitor milk flow during breast feeding sessions. Overall milk flow volumes may also be measured using a nipple shield with an integrated flow sensor.

There are many situations in which expressing breastmilk is useful and important to enable a mother to initiate or continue breastfeeding.

Expressing milk is for example useful to:
prevent leaking, relieve engorgement, blocked duct or milk stasis;
feed a baby while he/she learns to suckle or has difficulty in coordinating suckling or refuses suckling;
keep up the supply of breastmilk when a mother or baby is ill (hence build up a milk storage);
leave breastmilk for a baby when his/her mother is separated from the baby e.g. to work;
avoid pain during breastfeeding.

An optimal expressing or feeding rhythm is fundamental for optimizing milk supply, milk production, minimizing the risk of breast engorgement and infections, improving comfort and for the overall experience.

The milk production is a demand-supply process and a consistent pumping schedule increases milk supply. If nursing or pumping is consistently decreased for several days, the overall milk supply will decrease and a decrease in pumped amounts can be observed. Increased nursing/pumping conversely results in a greater milk supply. However, introducing extra pumping sessions between feeding is not always possible, and pumping too frequently can increase the risk of developing mastitis.

When directly breastfeeding the baby, the rhythm can be either established based on a schedule, on demand hence following the baby's cue, or at any other timing. If fed on demand, babies can easily adjust their mother's milk production by simply changing their breastfeeding length and frequency. However, for breastfeeding mothers using feeding schedules, or for mothers who alternate breastfeeding and pumping (e.g., pump during the week/day when at work and breastfeed during the evening/weekend) or for exclusively pumping mothers, breastfeeding tends to be a bumpier process.

Many mothers struggle to find the right rhythm, and they often seek help from clinicians when their milk production slows, or they may ask for help in preventing milk production from slowing over the long term. When they find their rhythm and desired comfort in breast feeding after much effort, often it is time to stop. Women frequently also declare that they do not feel empowered or confident in breastfeeding.

Especially first time mothers suffer from the loss of their own identity and impact that having a baby and breastfeeding has on their daily life, from what they can wear, limitations on what they can do and how they feel about their body and many mothers exhausted.

Among women who breastfeed for longer than three months, one of the most important reasons for weaning is returning to work. Mothers working as employees, full-time or part-time, have a higher risk of earlier breastfeeding cessation than non-working mothers. A key challenge contributing to the early cessation of breastfeeding among working mothers is the conflict with the work schedule.

Systems are known that focus on predicting the milk production and needs, or tracking the progress of breastfeeding and pumping.

There is a need for a system which can assist a mother in scheduling milk production in order to achieve an optimal rhythm, in particular to find a desired compromise between maximizing breastmilk production and ensuring comfort and convenience.

### SUMMARY OF THE INVENTION

The invention is defined by the claims.

According to examples in accordance with an aspect of the invention, there is provided a planning system for planning breast feeding or milk expression, comprising:
an input for receiving:
   first milk flow information from breast pumping sessions, comprising at least the time and milk volume of each breast pumping session; and
   second milk flow information from breast feeding sessions, comprising at least the time and milk volume for each breast feeding session; and
a processor for processing the first and second milk flow information to derive a schedule, wherein the schedule specifies a combination of future breast pumping and breast feeding sessions.

The "time" is intended to indicate the point in time, rather than the duration. The first and second information may however additionally indicate the duration of each respective session.

The first and second information defined above represent the minimum data needed to derive the schedule, but additional data may be used. This additional data is defined below as further information which may be provided to the "input". The input information may be combined into one or more actual data streams and hence there maybe one or more physical input paths to the processor.

This planning system monitors milk production during breast feeding e.g. using a nipple shield with an integrated flow sensor, and during expression using a breast pump. Using this historical information, a schedule is generated which specifies when future pumping and breast feeding should take place.

The first information may for example be obtained from monitoring sensors of a breast pump. The second information may for example be obtained from a monitoring system using an acoustic or optical flow sensor arrangement for application to the breast for monitoring milk flow levels. A known flow sensor arrangement for example comprises an ultrasound sensor and flow levels may be obtained by performing Doppler ultrasound processing.

The schedule aims to achieve an optimal milk production rhythm, for example finding a balance between maximizing breastmilk production and ensuring comfort and convenience. The system can promote a consistent rhythm for optimal milk production. It may therefore help mothers to find their rhythm early in their breastfeeding journey so that the experience of breastfeeding becomes one of pleasure ultimately supporting mother in maintaining breastfeeding their babies for longer.

The system may address the issues of uncertainties and worries of the user relating to the optimal rhythm for milk production, the perception of breastfeeding disrupting the daily life of the user, conflicts with personal and working life, and the mental load and stress (e.g. relating to the need to plan milk expression or breastfeeding).

The milk flow information may include additional information such as the quality of the expression or feeding session.

The information provided to the input may further comprise a desired milk production objective, comprising one of stockpiling, maximizing baby growth, achieving a consistent rhythm, reducing night time expression or feeding, or achieving breast emptying.

The schedule may in this way be derived taking into account a desired aim of the user at that particular time.

The processor for example uses machine learning to prepare the schedule. The use of machine learning (artificial intelligence) models and algorithms ensures a high level of personalization and adaptation to individual needs and circumstances of the mother and child.

The schedule for example specifies the duration of the breast pumping and breast feeding sessions, for example separately for each breast. Thus, the schedule indicates when and for how long feeding or expression should take place.

The information provided to the input may further comprise a user diary schedule. In this way, the schedule derived by the system may be matched to the user's diary so that the timings are matched to the most suitable timings for the user. This may for example take account of the time the user is at work or doing other activities. This means that breastfeeding can be made to fit better into the mother's daily life.

The information provided to the input may further comprise location information such that the first milk flow information and the second milk flow information each include associated location information, and wherein the schedule further specifies a location for each breast pumping and breast feeding session. The location may again be linked to the user's diary.

The information provided to the input may further comprise validation information indicating the extent to which the schedule has been followed. This feedback information enables the system to have more accurate knowledge of the feeding or expression (e.g. the amount of time) that has actually taken place. The validation information may be provided by the user or it may be automated, for example a breast pump may report the time and duration of breast pumping.

The processor is for example adapted to inform the user about an upcoming milk expression or feeding session by means of a notification. This assists the user in following the schedule. The user may be able to adjust the schedule for example to organize her activities based on the system predictions (e.g. system suggest pumping at 15:00h, but the user would like to start 30 minutes earlier).

The information provided to the input may further comprise user information comprising at least age and relevant health conditions and baby information comprising at least age, weight and length. The schedule may thus be adapted to the characteristics of the mother and/or the development of the baby and/or the amount of milk needed by the baby.

The information provided to the input may further comprise one or more of:
user and baby sleep tracking information and activity information for the user; a stress level indication for the user; and
dietary intake information for the user.

Sleep tracking can be used for detecting the sleep architecture of the baby (e.g. using a video camera, sleep mattress, etc.) and mother (e.g. using a wrist worn device, sleep mattress, etc.). A user-entered sleep diary may be used in place of an objective sleep tracker. It may for example provide sleep stage information as well derived sleep features such as sleep duration, sleep efficiency, number of awakenings per nights, etc.

A stress level can be provided by an external device for example using a measurement of heart rate, heart rate variability or skin conductance. Stress may be subjectively self-reported by the user (e.g. by periodic questionnaire).

The activity information for example relates to the amount of aerobic activity and the dietary intake information may involve self-reporting of food and caffeine intake (amount and time).

The information provided to the input may further comprise one or more of:
user weight tracking information;
baby weight tracking information; and
milk weight or volume tracking information.

This enables the schedule to adapt overtime to the changing circumstances.

The processor may be further adapted to suggest behavioral changes to improve milk production. These behavioral changes or interventions are for example to increase the feeding/milk expression duration, increase sleep time, increase physical activity, exchange nipple with regularity while breast feeding, expose baby to light during the day, reduce formula milk etc.

The planning system may further comprise one or more of:
a breast pump having a milk flow sensor arrangement;
a GPS location system;
a flow sensor arrangement for monitoring milk flow during breast feeding;
a user interface for allowing entry of user date to the system and output of the schedule; and
a scale for monitoring a weight of the baby or the mother or the milk produced during expression.

Thus, the required sensors may be part of the overall system.

The invention also provides a computer-implemented method for planning breast feeding or milk expression, comprising:
receiving first milk flow information from breast pumping sessions, comprising at least the time and milk volume of each breast pumping session;
receiving second milk flow information from breast feeding sessions, comprising at least the time and milk volume for each breast feeding session;
processing the first and second milk flow information to derive a schedule, wherein the schedule specifies a combination of future breast pumping and breast feeding sessions.

The schedule for example specifies the duration of the breast pumping and breast feeding sessions, for example separately for each breast.

The method may comprise receiving a desired milk production objective, comprising one of stockpiling, maximizing baby growth, achieving a consistent rhythm, reducing night time expression or feeding, or achieving breast emptying, and deriving a schedule takes account of the milk production objective.

The invention also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a planning system for planning milk expression or feeding; and
Figure 2 shows a method of planning milk expression or feeding.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a planning system for planning breast feeding or milk expression. An input receives first milk flow information from breast pumping sessions and second milk flow information from breast feeding sessions. The first and second milk flow information are processed to derive a schedule, wherein the schedule specifies a combination of future breast pumping and breast feeding sessions. In this way, the system assists a mother in planning her milk production routine to best fit with her schedule as well as providing the best nutrition for the infant.

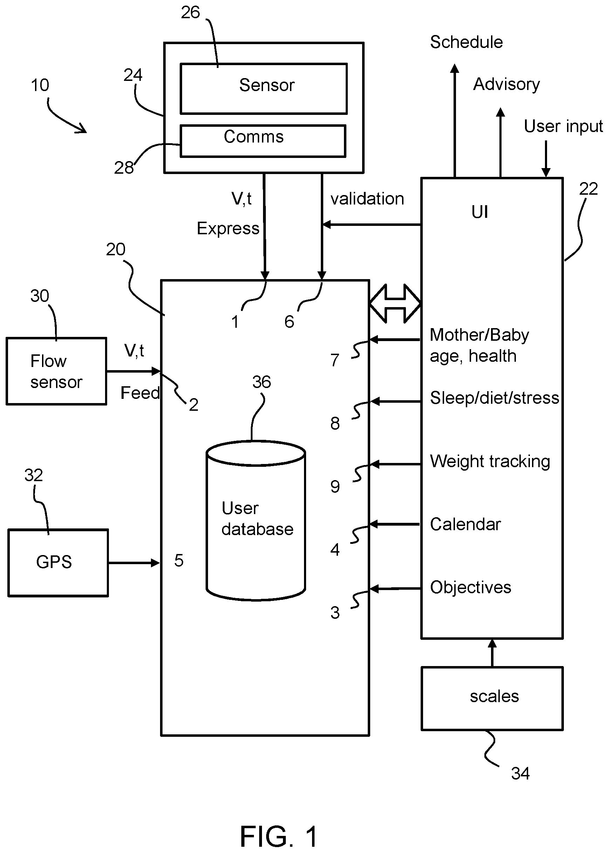

Figure 1 shows a planning system 10 for planning breast feeding or milk expression. The system comprises a processor 20 which receives various input information in order to derive an output to a user, by means of a user interface 22, in the form of a schedule which includes breast feeding times and expression times. The various types of input information are shown as separate physical inputs to the processor in Figure 1, simply for the purpose of explanation. In practice, multiple signals may be combined before being provided to one or more data inputs of the processor.

The processor may be located on a mobile phone or on a remote server and the system output can be conveyed to the user by sound (e.g. by a digital assistant), by a display, such as charts shown via a mobile/desktop app, or by text messages etc.

The physical inputs to the processor are numbered 1 to 9 in Figure 1 for the purposes of explanation, each representing a different type of information to be processed by the processor. As a minimum, there is a first input for receiving first milk flow information from breast pumping sessions, comprising at least the time t (e.g. the point in time at which the session started) and milk volume V of each breast pumping session.

This first milk flow information is for example collected by a breast pump 24 having a milk flow sensor 26 and a communications module 28 for communicating with the processor 20. The breast pump may provide information about the pumping sessions: time, flow rate, milk quantity.

A second input is for receiving second milk flow information from breast feeding sessions, comprising at least the time and milk volume for each breast feeding session.

The second milk flow information is for example collected by a milk flow sensor used during feeding, or it may be reported by the mother via the user interface (e.g. reporting the time of feeding so that an estimate of volume may be made).

This information may be delivered by a flow (and timing) sensor 30 which may for example be integrated into a breast shield used during breast feeding. The milk flow sensor 30 for feeding may comprise part of a so-called smart nipple which provides information about the breast feeding session: duration per nipple and milk expressed quantity. The sensor may be an acoustic or optical flow sensor arrangement for application to the breast for monitoring milk flow levels. A known flow sensor arrangement for example comprises an ultrasound sensor and flow levels may be obtained by performing Doppler ultrasound processing.

The processor 20 processes the various inputs, namely at least the first and second milk flow information, to derive the schedule, which is output by the user interface 22. The processor for example uses machine learning to prepare the schedule with a high level of personalization and adaptation to individual needs and circumstances of the mother and child. The milk flow information may include additional information such as the quality of the expression or feeding session.

The planning system thus monitors milk production during breast pumping and during expression using a breast pump. Using this historical information, a schedule is generated which specifies when future pumping and breast feeding should take place. The schedule for example specifies the duration of the breast pumping and breast feeding sessions, for example separately for each breast. Thus, the schedule indicates when and for how long feeding or expression should take place. The processor may inform the user about an upcoming milk expression or feeding session by means of a notification.

This may be provided as part of advisory information also output from the user interface 22. This assists the user in following the schedule. The user may able to adjust the schedule for example to organize her activities, using the user interface.

For example, when a notification is provided, the mother can accept the schedule and start a pumping or feeding session, select a snooze option to postpone pumping or feeding session and indicate a reason, or select a decline option to skip the pumping or feeding session and indicate reason.

The schedule aims to achieve an optimal milk production rhythm, for example finding a balance between maximizing breastmilk production and ensuring comfort and convenience. The system can promote a consistent rhythm for optimal milk production. It may therefore help mothers to find their rhythm early in their breastfeeding journey so that the experience of breastfeeding becomes one of pleasure ultimately supporting mother in maintaining breastfeeding their babies for longer.

The schedule may provide an indication of future milk expression and breastfeeding in terms of the timing, duration, quantity and location.

The system may also provide as output suggested behavioral changes to improve milk production, as part of the advisory information output shown in Figure 1. These behavioral changes or interventions are for example to increase the feeding/milk expression duration, increase sleep time, increase physical activity, exchange nipple with regularity while breast feeding, expose baby to light during the day, reduce formula milk etc.

The recommendations may take account of the user's sleep, their eating habits, personal schedule (as explained further below) and they may relate to drinking, eating or sleeping recommendations.

The suggested behavioral changes or interventions are for example:
Increase feeding/milk expression duration;
Increase sleep time;

Improve sleep quality (e.g. reduce awakenings, increase deep sleep duration, reduce sleep onset latency);
Increase physical activity;
Exchange nipple with regularity while breast feeding;
Expose baby to light during the day;
Engage in paced breathing exercise when feeling stressed;
Begin and end the day with a mindfulness exercise;
Reduce formula milk.

Some of these recommendations are derived from the additional inputs as explained below. The system may also provide positive motivational feedback.

Figure 1 shows a third input for receiving a desired milk production objective. Various different objectives may be set such as stockpiling, maximizing baby growth, achieving a consistent rhythm, reducing night time expression or feeding, or achieving breast emptying. The desired objective is for example input to the user interface 22 as user input.

Figure 1 shows a fourth input for receiving a user diary schedule i.e. a user calendar. In this way, the schedule derived by the system may be matched to the user's diary so that the timings are matched to the most suitable timings for the user. This may for example take account of the time the user is at work or doing other activities. This means that breastfeeding can be made to fit better into the mother's daily life. The calendar input is for example input to the user interface 22 as user input. It may include personal and professional commitments and priorities.

Figure 1 shows a fifth input for receiving location information from a location tracking system such as a GPS system 32, such that the first milk flow information and the second milk flow information are each linked to associated location information. The schedule may then further specify a location for each breast pumping and breast feeding session. The location may again be linked to the user's diary.

Figure 1 shows a sixth input for receiving validation information indicating the extent to which the schedule has been followed. The validation information may be received from the user interface 22 where it has been provided as user input or it may be derived from the sensor information of a breast pump, in the case of an expression session. The validation information indicates whether the previous session(s) were according to the planner or spontaneous (due to discomfort/ baby crying). The validation information may include the time and duration of a pumping and feeding session.

Figure 1 shows a seventh input for receiving user information comprising at least age and relevant health conditions of the mother (e.g. nipple condition, number of pregnancies) and baby information comprising at least age, weight and length. The schedule may thus be adapted to the characteristics of the mother and the development of the baby. The calendar input is for example input to the user interface 22 as user input. The user may make use of scales 34 for this purpose.

Figure 1 shows an eighth input for receiving one or more of:
user and baby sleep tracking information and activity information for the user; a stress level indication for the user; and
dietary intake information for the user.

Sleep tracking can be used for detecting the sleep architecture of the baby (e.g. using a video camera, sleep mattress, etc.) and mother (e.g. using a wrist worn device, sleep mattress, etc.). A user-entered sleep diary may be used in place of an objective sleep tracker. It may for example provide sleep stage information as well derived sleep features such as sleep duration, sleep efficiency, number of awakenings per nights, etc.

A stress level can be provided by an external device for example using a measurement of heart rate, heart rate variability or skin conductance. Stress may be subjectively self-reported by the user (e.g. by periodic questionnaire).

The activity information for example relates to the amounts of aerobic activity and the dietary intake information may involve self-reporting of food and caffeine intake (amount and time). The activity tracker for example provides the number of minutes that the user has engaged in an aerobic activity (with aerobic activity being characterized by heart rate level between 55% and 85% of the users' maximum heart rate, with maximum heart rate calculated 207 - 0.7^{∗}age).

Figure 1 also shows a ninth input for receiving one or more of:
user weight tracking information;
baby weight tracking information; and
milk weight tracking information.

Again some of this information may be measured by the user by means of the scales 34.

As will be clear from the options above, the system provides predictions of the best times (and quantities) for milk expression and/or breast feeding based on historical data for the subject. It can also provide recommendations for improving the milk expression experience and efficiency based on the mother and baby's physical characteristics, sleep schedule, habits, stress level and baby growth.

The schedule and recommendations can also take account of other user data with similar characteristics. Targets can change over time or even not be defined.

Examples of recommendations are for example a recommendation to increase frequency of expression in response to a detected low milk expression quantity.

Figure 1 shows a user database 36, which may store prior pumping and feeding sessions information as well as the user information.

The user interface may additionally be used to give feedback from the user in respect of the suitability of the proposed schedule. For example if the schedule was not followed, the reason may be provided, for example the need for a spontaneous feed due to discomfort or the baby crying. The user may give a rating of the schedule.

The data in the database may be interrogated to provide a report of daily or monthly data overviews, patterns and trends to indicate what to expect in terms of expressed milk quantity, feeding/pumping frequency, or the need for cluster feeding (for a growth spurt).

The system may additionally store historical data relating to bottle feeding sessions. For example, a so-called smart feeding bottle may provide information about bottle feeding time and quantity.

Another possible sensor input is a wearable sensor for light exposure detection, to provide the duration, intensity and timing of both sunlight and artificial light detected up to the users' bedtime. It can be implemented as a button put the clothing of the baby and mother.

The schedule generated by the processor for example proposes milk expression daily sessions based on the various inputs discussed above taking into account the user's target and optionally their location. The user can accept/edit/discard specific or all sessions proposed in the schedule. The location for feeding or expression may be proposed based on GPS data (e.g. it may be determined if a supermarket has a feeding room and propose it as location).

The processor may also flag conflicts between the desired schedule and the calendar information and then offer options. For example, if a conflicting session is meant to keep the rhythm of the baby drinking scheme, and this rhythm has been disrupted already for another day, this session may have a higher priority. Conversely if an extra session was planned for storage a different priority may be set.

Table 1 below shows an example of a milk expression schedule proposed by the processor.

**Table 1**

| **Start Time** | **Event type** | **Duration** | **Location** |
|---|---|---|---|
| 6:00 | Breast feeding | 10 mins right. & 10 mins left | home |
| 9:00 | Pumping | 10 mins right. & 10 mins left | office |
| 12:00 | Pumping | 10 mins right. & 10 mins left | office |
| 15:00 | Pumping | 10 mins right. & 10 mins left | feeding room of grocery store |
| 18:00 | Breast feeding | 10 mins right. & 10 mins left | home |

Table 2 shows an example of the collected "raw" data relating to expression sessions.

**Table 2**

| **Date** | **Time** | **Time of Day** | **Quantity (ml)** | **Duration (mins)** | **Location** | **Event** | **Weight (kg)** | **Length(cm)** | **Week** |
|---|---|---|---|---|---|---|---|---|---|
| 20200101 | 24 | night | 600 | 10 | home | feeding | 3 | 51 | 1 |
| 20200101 | 3 | night | 550 | 5 | home | feeding | 3 | 51 | 1 |
| 20200101 | 6 | morning | 700 | 5 | home | feeding | 3.1 | 51 | 1 |
| 20200101 | 10 | morning | 800 | 15 | office | pumping | 3.1 | 51 | 1 |
| 20200101 | 13 | afternoon | 850 | 16 | office | pumping | 3.1 | 51 | 1 |
| 20200101 | 16 | afternoon | 700 | 12 | office | pumping | 3.1 | 51 | 1 |
| 20200101 | 19 | night | 800 | 10 | home | feeding | 3.1 | 51 | 1 |
| 20200101 | 21 | night | 650 | 10 | home | pumping | 3.2 | 51 | 1 |
| 20200102 | 1 | night | 600 | 5 | home | pumping | 3.2 | 51 | 2 |
| 20200102 | 5 | night | 700 | 7 | home | feeding | 3.2 | 51 | 2 |
| 20200102 | 8 | morning | 800 | 11 | home | feeding | 3.2 | 51 | 2 |
| 20200102 | 11 | morning | 850 | 14 | office | pumping | 3.2 | 53 | 2 |
| 20200102 | 14 | afternoon | 700 | 5 | office | pumping | 3.2 | 53 | 2 |
| 20200102 | 15 | afternoon | 750 | 10 | office | pumping | 3.2 | 53 | 2 |
| 20200102 | 18 | afternoon | 650 | 15 | office | pumping | 3.25 | 53 | 2 |
| 20200102 | 22 | night | 600 | 10 | home | breast | 3.25 | 53 | 2 |
| 20200102 | 24 | night | 650 | 10 | home | breast | 3.25 | 53 | 2 |

Table 3 shows an example of the collected "raw" data relating to breast feeding.

**Table 3**

| **Date** | **Time** | **Time of** | **Quantity** | **Duration** | **Location** | **Mode** | **Weight** | **Length** | **Week** |
|---|---|---|---|---|---|---|---|---|---|
| 20200101 | 24 | night | 600 | 10 | home | breast | 3 | 51 | 1 |
| 20200101 | 3 | night | 550 | 5 | home | breast | 3 | 51 | 1 |
| 20200101 | 6 | morning | 700 | 5 | home | breast | 3.1 | 51 | 1 |
| 20200101 | 10 | morning | 800 | 15 | gradma | bottle | 3.1 | 51 | 1 |
| 20200101 | 13 | afternoon | 850 | 16 | gradma | bottle | 3.1 | 51 | 1 |
| 20200101 | 16 | afternoon | 700 | 12 | gradma | bottle | 3.1 | 51 | 1 |
| 20200101 | 19 | night | 800 | 10 | home | breast | 3.1 | 51 | 1 |
| 20200101 | 21 | night | 650 | 10 | home | bottle | 3.2 | 51 | 1 |
| 20200102 | 1 | night | 600 | 5 | home | bottle | 3.2 | 51 | 2 |
| 20200102 | 5 | night | 700 | 7 | home | breast | 3.2 | 51 | 2 |
| 20200102 | 8 | morning | 800 | 11 | home | breast | 3.2 | 51 | 2 |
| 20200102 | 11 | morning | 850 | 14 | park | bottle | 3.2 | 53 | 2 |
| 20200102 | 14 | afternoon | 700 | 5 | gradma | bottle | 3.2 | 53 | 2 |
| 20200102 | 15 | afternoon | 750 | 10 | gradma | bottle | 3.2 | 53 | 2 |
| 20200102 | 18 | afternoon | 650 | 15 | gradma | bottle | 3.25 | 53 | 2 |
| 20200102 | 22 | night | 600 | 10 | home | breast | 3.25 | 53 | 2 |
| 20200102 | 24 | night | 650 | 10 | home | breast | 3.25 | 53 | 2 |

Data pushing is for example triggered by event occurrence (i.e. a new expression or feeding event, or user interaction with the system user interface) and data are accumulated over time.

The raw data are for example processed with a time moving window (different time window lengths can be used such as 1 day for daily statistics, 7 days for weekly statistics) in order to extract time-dependent statistical features. Examples of features of interest are:
Number of feedings per night;
Averaged milk intake per morning/afternoon/night;
Variation of milk intake per morning/afternoon/night;
Averaged milk expressed per morning/afternoon/night;
Variation of milk expressed per morning/afternoon/night;
Cumulative milk intake per day;
Cumulative milk intake per week;
Cumulative milk expressed per day;
Cumulative milk expressed per week;
Baby weight change per week;
Baby length change per week;
Cumulative expressed milk based on location (home/workplace/others);
Difference between expressed and used milk per day;
Time difference between milk expression sessions; and
Time difference between feeding sessions.

Any suitable mathematical aggregation of the variables in Table 2 and Table 3, over any time window range (hourly, daily, weekly, etc.) may be used as an input for making predictions and providing recommendations.

The distributions and comparison between the distributions of certain variables may be used to obtain insights about behaviors and generate the recommendations. Such distributions may for example show that the quantity of milk expressed by pumping is higher than by breast feeding and that the quantity of milk expressed during the night is lower than during the day. From such insights, it can be recommended to increase the breast-feeding time.

The comparison between variable distributions and a quantification of statistical differences between such distributions can be calculated by applying statistical tests according to the variable type and distribution (e.g. t-test for continuous variables characterized by a normal distribution).

The raw data as well as the statistical features are used to provide personalized prediction models on which the schedule is based. The prediction models are generated using machine learning. The variables to include in each prediction model can be selected in different ways. For example, variables may be chosen having a correlation coefficient with a target variable above as threshold. A variable selection procedure can also be embedded in the training of the models.

The choice of the model type will be driven by the amount of available data, the output variables (e.g. time of the next feeding session, quantity of daily expressed milk), the learning time for the model parameters and the computational power of the processing unit. Some of the models which could be used are the Exponential Smoothing, ARIMA, Decision Tree Regressor, Support Vector Regressor, Lasso linear model, Generalized linear model. Model parameters can be estimated by minimization of the Mean Squared Error, but any other estimation approach can be applied.

In order to account for changing of baby and mother's characteristics' and needs, models can be retrained using a fixed schedule or when an accuracy of the model output decreases.

If there is missing data for a particular user, so that the model training has insufficient data, data of similar users (so-called twin users) or models estimated for such twin users can be used instead. Twin users can be found by using clustering algorithms (e.g. K-Means). These will be used to divide users (mother and baby coupled as one unit) in clusters based on their features (e.g. baby age, mother age, mother job, baby sleep cycle etc.). Some or all subjects falling in the same cluster of the specific user can then be selected as twin users. Such twin users can be ranked based on a statistical distance (or variable space geometric distance) calculated from the specific user; a high rank (similarity) will be given to the user which has the lowest distance from the specific user. The Euclidean distance can be used for calculating the users' ranking.

The output of different prediction models may be directly shown to the user (e.g. time of next feeding) and also be used by the processor to generate an optimal schedule and recommendations.

The system may be used to provide a schedule for optimizing baby growth according to the baby's age, length, gender and birth weight. The processor may for example compare the baby weight with the population weight (obtained from literature) and calculate differences from such weight at a specific week/month. In the case of under or overweight, the system may use characteristics of the feeding sessions, baby and mother behaviors and habits (e.g. low feeding frequency rate at night, low feeding duration, limited sleep duration) for recommending a feeding schedule which will improve the rate of increase of the baby's weight. The schedule can be shared with a pediatrician for obtaining approval and further advice.

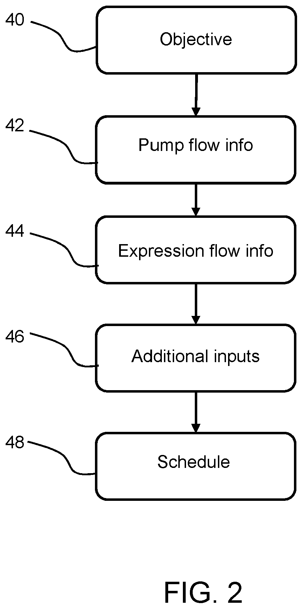

Figure 2 shows a method for planning breast feeding or milk expression.

In step 40, a desired milk production objective is input, comprising one of stockpiling, maximizing baby growth, achieving a consistent rhythm, reducing night time expression or feeding, or achieving breast emptying.

In step 42, first milk flow information is received from breast pumping sessions, comprising at least the time and milk volume of each breast pumping session.

In step 44, second milk flow information from breast feeding sessions, comprising at least the time and milk volume for each breast feeding session.

In step 46, one or more optional additional inputs are received as explained above.

In step 46, the various inputs, i.e. at least the first and second milk flow information, are processed to derive a schedule which specifies a combination of future breast pumping and breast feeding sessions, in particular to meet the objective set.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

The processor may be the processor of a mobile phone on which a suitable app has been loaded, or the processor may be remote and the local user device (such as a mobile phone or app) only serves to collect data from the remote processor for presentation e.g. display, to the user. Thus, the various processing tasks (sensor data collection, user input, schedule preparation, historical database access etc.) may be split between different locations in any desired manner.

A computer program may be stored/distributed on a suitable medium, such as an optical storage medium or a solids state medium supplied together with or as part of other hardware, but may also be distributed in other forms, such as via the Internet or other wired or wireless telecommunication systems.

The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". Any reference signs in the claims should not be construed as limiting the scope.

## Claims

1. A planning system (10) for planning breast feeding or milk expression, comprising:
an input (1-9) for receiving:
first milk flow information from breast pumping sessions, comprising at least the time and milk volume of each breast pumping session; and
second milk flow information from breast feeding sessions, comprising at least the time and milk volume for each breast feeding session; and
a processor (20) for processing the first and second milk flow information to derive a schedule, wherein the schedule specifies a combination of future breast pumping and breast feeding sessions.

2. The planning system of claim 1, wherein the input is further for receiving a desired milk production objective, comprising one of stockpiling, maximizing baby growth, achieving a consistent rhythm, reducing night time expression or feeding, or achieving breast emptying, and the processor (20) is further for processing the objective to derive the schedule.

3. The planning system of claim 1, wherein the processor (20) uses machine learning to prepare the schedule.

4. The planning system of any one of claims 1 to 3, wherein the schedule specifies the duration of the breast pumping and breast feeding sessions, for example separately for each breast.

5. The planning system of any one of claims 1 to 3, wherein the input is further for receiving a user diary schedule, and the processor (20) is further for processing the user diary schedule to derive the schedule.

6. The planning system of any one of claims 1 to 5, wherein the input is further for receiving location information such that the first milk flow information and the second milk flow information each include associated location information, and wherein the processor (20) is further for processing the location information, and the schedule further specifies a location for each breast pumping and breast feeding session.

7. The planning system of any one of claims 1 to 6, wherein the input is further for receiving validation information indicating the extent to which the schedule has been followed.

8. The planning system of any one of claims 1 to 7, wherein the processor (20) is adapted to inform the user about an upcoming milk expression or feeding session by means of a notification.

9. The planning system of any one of claims 1 to 8, wherein the input is further for receiving user information comprising at least age and relevant health conditions and baby information comprising at least age, weight and length and the processor (20) is further for processing the user and baby information to derive the schedule.

10. The planning system of any one of claims 1 to 9, wherein the input is further for receiving one or more of:
user and baby sleep tracking information and activity information for the user; a stress level indication for the user; and
dietary intake information for the user.

11. The planning system of any one of claims 1 to 10, wherein the input is further for receiving one or more of:
user weight tracking information;
baby weight tracking information; and
milk weight or volume tracking information.

12. The planning system of any one of claims 1 to 11, wherein the processor (20) is further adapted to suggest behavioral changes to improve milk production.

13. The planning system of any one of claims 1 to 12, further comprising one or more of:
a breast pump (24) having a milk flow sensor arrangement (26);
a GPS location system (32);
a flow sensor arrangement (30) for monitoring milk flow during breast feeding;
a user interface (22) for allowing entry of user date to the system and output of the schedule; and
a scale (34) for monitoring a weight of the baby or the mother or the milk produced during expression.

14. A computer-implemented method for planning breast feeding or milk expression, comprising:
(42) receiving first milk flow information from breast pumping sessions, comprising at least the time and milk volume of each breast pumping session;
(44) receiving second milk flow information from breast feeding sessions, comprising at least the time and milk volume for each breast feeding session;
(46) processing the first and second milk flow information to derive a schedule, wherein the schedule specifies a combination of future breast pumping and breast feeding sessions.

15. A computer program comprising computer program code means which is adapted, when said program is run on a computer, to implement the method of claim 14.
